# EUROPÄISCHE PATENTSCHRIFT

(11) **EP 2 120 696 B1**
(45) Veröffentlichungstag und Bekanntmachung des Hinweises auf die Patenterteilung: **22.06.2011**
(21) Anmeldenummer: 08716943.9
(22) Anmeldetag: 19.02.2008
(51) Int. Cl.: A61B 5/0492

(54) **STAPEDIUSMUSKELELEKTRODE**
STAPEDIUS MUSCLE ELECTRODE
ÉLECTRODE POUR LE MUSCLE DE L'ÉTRIER

(30) Priorität: 19.02.2007 DE 102007008154
(43) Veröffentlichungstag der Anmeldung: 25.11.2009
(73) Patentinhaber: MED-EL Elektromedizinische Geräte GmbH, 6020 Innsbruck (AT)
(72) Erfinder: PAU, Hans, Wilhelm, 18055 Rostock (DE); BEHREND, Detlef, 18119 Warnemünde (DE); SCHMIDT, Wolfram, 18109 Rostock (DE); SCHMITZ, Klaus-Peter, 18119 Warnemünde (DE)
(74) Vertreter: Lucke, Andreas
(86) Internationale Anmeldenummer: PCT/EP2008/051995
(87) Internationale Veröffentlichungsnummer: WO 2008/101922

(56) Entgegenhaltungen:
- US-A- 4 573 481
- US-B1- 6 208 882

## Beschreibung

Die vorliegende Erfindung betrifft eine Stapediusmuskelelektrode zur Befestigung an einem menschlichen Stapediusmuskel und zur Detektion der bei einer Kontraktion des Stapediusmuskels erzeugten Aktionsströme.

Im Mittelohr eines Menschen befinden sich die drei Gehörknöchelchen Hammer, Amboß und Steigbügel, die die Ankopplung des Trommelfells an das Innenohr bewirken. Dazu steht der Hammer mit der Innenseite des Trommelfells in Verbindung, während der Steigbügel mit dem ovalen Fenster des Innenohres in Verbindung steht. Auf diese Weise kann der Hammer die Schwingungen des Trommelfells aufnehmen und über den zwischen Hammer und Steigbügel angeordneten Amboß und den Steigbügel auf das ovale Fenster übertragen, wodurch in der Flüssigkeit der Gehörschnecke Schallschwingungen erzeugt werden, die die Flüssigkeit durchlaufen. Diese Schallschwingungen versetzen in die Flüssigkeit hineinragende Haarzellen in Bewegung, und die Bewegung der Haarzellen löst Nervenimpulse aus, die schließlich zur Erzeugung eines Höreindrucks an das Gehirn weitergeleitet werden.

Im Normalfall ist es erwünscht, daß möglichst die gesamte im Gehörgang eintreffende Schalleistung über die Gehörknöchelchen an das Innenohr weitergegeben wird. Ab einem bestimmten Schalldruck würde die ungedämpfte Schallweiterleitung jedoch zu einer Schädigung des Innenohres führen. Aus diesem Grunde ist im menschlichen Ohr ein Mechanismus vorgesehen, mit dem die Kette der Gehörknöchelchen in ihrem Schwingungsverhalten beeinflußt und die durch sie bereitgestellte Schalleitung bei starken akustischen Ereignissen herabgesetzt werden kann. Dieser natürliche Schutzmechanismus basiert auf zwei kleinen Muskel, die sich bei Wahrnehmung eines hohen Schallpegels unwillkürlich reflexartig anspannen. Dabei setzt der sogenannte Trommelfellspannmuskel am Hammer an und spannt das Trommelfell, während der sogenannte Stapediusmuskel über eine ihm zugeordnete Sehne am Steigbügel angreift und die Fußplatte des Steigbügels im ovalen Fenster verkantet. Auf diese Weise wird die Kette der Gehörknöchelchen versteift, so daß sich die Ankopplung des Trommelfells an das Innenohr verschlechtert. Ein Teil der am Trommelfell eintreffenden Schalleistung wird am Trommelfell reflektiert und ein weiterer Teil wird in die umliegenden Knochen abgeleitet. So wird das Gehör in gewissen Grenzen vor einer Schädigung durch zu hohe Schalldrücke geschützt.

Dieser in der Anspannung des Stapediusmuskels resultierende Reaktionsmechanismus des Gehörs zum Schutze des Innenohrs vor Schäden durch zu hohe Schallpegel wird als Stapediusreflex bezeichnet. Er setzt normalerweise bei Schallpegeln von über etwa 80 bis 100 dB ein und tritt ungefähr 50 ms nach Beginn der Schalleinwirkung auf. Der Stapediusreflex ist in der Medizin von Bedeutung, da aus einem untypischen Verhalten oder seinem vollständigen Fehlen Rückschlüsse auf die Funktionsfähigkeit des Ohres gezogen werden können. So kann der Einsatz des Reflexes bei relativ zu geringen oder relativ zu hohen Schallpegeln eine Funktionsstörung des Ohres anzeigen, während sein Fehlen zum einen auf einen z.B. innenohrbedingten Verlust des Gehörs hindeuten kann und zum anderen - und das ist aus klinischer Sicht bedeutsamer - auf eine sogenannte Otosklerose, d.h. eine Schalleitungsschwerhörigkeit auf Grund einer Fixierung des Steigbügels.

Darüber hinaus ist die Beobachtung des Auftretens des Stapediusreflexes in Zusammenhang mit Cochleaimplantaten von großer praktischer Bedeutung, die bei tauben Menschen eingesetzt werden, deren Taubheit durch das Fehlen oder die Zerstörung der Haarzellen in der Gehörschnecke verursacht wird. Diese Form der Taubheit kann nicht durch konventionelle Hörgeräte überwunden werden, die lediglich eine Verstärkung der durch das Ohr empfangenen Schallwellen herbeiführen. Vielmehr müssen die an das Gehirn weiterzuleitenden elektrischen Nervenimpulse künstlich erzeugt werden, was durch in die Gehörschnecke implantierte Stimulationselektroden erreicht werden kann, die einen Teil des Cochleaimplantats bilden. Durch Cochleaimplantate kann bei intaktem Hörnerv das Hörvermögen vielfach zumindest teilweise so wiederhergestellt werden, daß ein Sprachverständnis möglich ist. Dabei ist es allerdings wichtig, daß die von den Stimulationselektroden abgegebenen Signalpegel in geeigneter Weise eingestellt und geregelt werden. Da aus dem Auftreten des Stapediusreflexes auf die von dem Patienten wahrgenommene Schallenergie geschlossen werden kann, kann die Messung des Stapediusreflexes zur passenden Einstellung des Cochleaimplantats benutzt werden. Bildet eine Sensoreinrichtung zur Erkennung des Stapediusreflexes einen integralen Teil des Cochleaimplantats, ist eine Selbstadaption von dessen Energieabgabe möglich.

Zur Messung des Stapediusreflexes werden unter anderem Elektroden eingesetzt, die mit dem Gewebe des Stapediusmuskels in Verbindung gebracht werden und auf diese Weise die bei einer Kontraktion des Stapediusmuskels erzeugten Aktionsströme aufnehmen und an eine geeignete Meßeinrichtung leiten können. Dabei hat sich jedoch die zuverlässige Kontaktierung des Stapediusmuskels als schwierig erwiesen, da er zum größten Teil innerhalb eines im Knochen verborgenen Kanals angeordnet ist und nur ein kleiner Teil von ihm und seine Sehne von dem Inneren des Mittelohrs aus sichtbar und ohne weiteres zugänglich sind.

Die Druckschrift US 6,208,882 beschreibt eine Reihe unterschiedlich ausgestalteter Elektroden zur Befestigung an dem Stapediusmuskelgewebe. Die Merkmale des Oberbegriffs von Anspruch 1 sind aus dieser Druckschrift bekannt.

Eine dieser Elektroden wird durch eine gezackte, flache Klinge gebildet, die durch eine chirurgisch hergestellte (Schlitz-) Inzesion in dem Stapediusmuskel geführt und dort durch die Widerhaken bildenden Zacken gehalten wird. Diese Elektrode hat den Nachteil, daß der erforderliche Einschnitt in den Muskel und das Vorhandensein von Widerhaken mit einer erheblichen Traumatisierung verbunden ist. Außerdem ist sie aufgrund der Widerhaken auch nur schlecht bzw. mit Komplikationen entfernbar. Alternativ kann eine derartige Elektrode auch befestigt werden, indem eine Bohrung durch den den knöchernen Kanal seitlich begrenzenden Knochen erzeugt, die Klinge durch die Bohrung geführt und in dem Kanal nach oben umgebogen wird, so daß sie zwischen der Innenwand des Kanals und dem Stapediusmuskel verläuft. Zur sichereren Befestigung kann die Klinge außerdem über den oberen Rand des Kanals umgebogen werden. Auch hierbei besteht jedoch der Nachteil einer Traumatisierung aufgrund der Knochenbohrung. Außerdem kommt es zu einer Impedanzerhöhung im Restknochen, und die Klinge ist bei der Befestigung nur schwer steuerbar, so daß auch die Gefahr einer direkten Traumatisierung des Muskels besteht.

Eine weitere dieser Elektroden umfaßt einen biokompatiblen Metalldraht, der flexibel oder in Form eines Hakens vorgeformt ist und der an einem Ende eine kleine Kugel aufweist und an dem anderen Ende mit einem gewendelten isolierten Draht zur Verbindung mit einer Auswerteschaltung verbunden ist. Bei der Implantation wird die Elektrode derart in den knöchernen Kanal eingeführt, daß die Kugel gegen den Stapediusmuskel gedrückt und zwischen diesem und dem angrenzenden Knochen festgeklemmt wird. Die richtige Anordnung dieser Elektrode erfordert Spezialinstrumente. Außerdem ist die Hakenform relativ raumfordernd, und die Befestigung ist nicht sehr sicher, so daß die Elektrode durch Muskelbewegungen leicht disloziert werden kann. Zudem ist gelegentlich die Entfernung eines Knochenstücks erforderlich.

Weitere dieser Elektroden sind so ausgebildet, daß sie um den aus dem knöchernen Kanal vorstehenden Abschnitt des Stapediusmuskels herum gelegt und gesichert werden können. So wird eine Elektrode durch eine um den Muskel klemmbare Silikonmanschette gebildet, auf deren Innenseite elektrische Kontakte ausgebildet sind. Eine andere Elektrode wird durch einen haken- oder U-förmig vorgeformten Draht gebildet, an dessen Enden sich kleine Kugeln oder Schlaufen befinden und der um den Stapediusmuskel gebogen und fixiert werden kann. Diese Ausgestaltungen sind nicht nur aufwendig in der Handhabung (z.B. erfordern sie ein Spreizungsinstrument), relativ raumfordernd und kompliziert aufgebaut, sondern ihr Einsatz birgt auch die Gefahr einer Drucknekrose in sich.

Noch eine andere dieser Elektroden wird durch einen mehradrigen, teflonisolierten Pt/Ir-Draht gebildet. Zur Befestigung in dem Muskelgewebe wird am vorderen Ende des Drahtes ein Stück der Isolierung entfernt und nach hinten umgebogen. Anschließend wird eine Bohrung durch den Knochen vorgenommen, durch die der Draht durch eine Nadel in den Muskelbauch eingeschoben wird. Bei Entfernung der Nadel wirkt das umgebogene Drahtvorderende als Widerhaken, die den Draht in dem Gewebe festhalten. Auch dies ist in nachteiliger Weise mit einer Traumatisierung verbunden.

Allgemein haben diese bekannten Stapediusmuskelelektrodenanordnungen somit die Nachteile, daß sie stark traumatisierend sind, Knochenbohrungen erfordern, nur mit Spezialinstrumenten zu implantieren sind und/oder eine unzureichende Fixierung bei Muskelbewegungen aufweisen sowie schwer oder gar nicht wieder entfernbar sind.

Es ist Aufgabe der vorliegenden Erfindung, eine einfach aufgebaute Stapediusmuskelelektrodenanordnung bereitzustellen, die ohne Verursachung von Dauerschäden zuverlässig am Stapediusmuskel fixiert werden kann und die die genannten Nachteile überwindet.

Zur Lösung dieser Aufgabe dienen die Merkmale von Patentanspruch 1. Vorteilhafte Ausführungsformen der Stapediusmuskelelektrodenanordnung sind Gegenstand der zugehörigen Unteransprüche.

Nach der vorliegenden Erfindung ist vorgesehen, daß eine Stapediusmuskelelektrodenanordnung eine für eine bipolare Ableitung angepaßte Elektrode zur Befestigung an einem menschlichen Stapediusmuskel umfaßt, wobei die Elektrode zwei biokompatible, flexible, elastische, elektrisch leitende längliche Elemente aufweist, von denen jedes ein distales und ein proximales Ende aufweist. Dabei bezeichnet der Begriff "proximal" in der vorliegenden Anmeldung in üblicher Weise das während des Befestigungsvorgangs der Bedienungsperson zugewandte Ende, während sich der Begriff "distal" in üblicher Weise auf das der Bedienungsperson abgewandte und dem Patienten zugewandte Ende bezieht. Dementsprechend ist das distale Ende der beiden länglichen Elemente dasjenige Ende, das bei der nachfolgend beschriebenen, bestimmungsgemäßen Elektrodenbefestigung zuerst mit dem Stapediusmuskel in Berührung kommt. Jedes der beiden länglichen Elemente ist entlang seiner gesamten Länge oder entlang eines Teils seiner Länge wendelförmig vorgeformt, wobei sich der auf diese Weise gebildete wendelförmige Teil oder Abschnitt jedenfalls bis zum distalen Ende des länglichen Elements erstreckt.

Im Rahmen der vorliegenden Anmeldung wird unter "wendelförmig" ein Verlauf eines länglichen Elements verstanden, bei dem das längliche Element sich um eine gerade, gekrümmte oder beliebig geformte gedachte Linie windet und dabei gleichzeitig der Richtung dieser Linie folgt. Die gedachte Linie, die die allgemeine Erstreckungsrichtung des wendelförmigen Verlaufs definiert, wird als Mittellinie des wendelförmigen Verlaufs bezeichnet. Mit anderen Worten ist der Verlauf derart, daß er ein Volumen wenigstens teilweise umschließt, dessen Querschnittsfläche senkrecht zur allgemeinen Erstreckungsrichtung des länglichen Elements verläuft. Dabei kann das umschlossene Volumen eine beliebige Querschnittsform aufweisen. Ein Beispiel eines wendelförmigen Verlaufs ist ein schraubenlinienförmiger Verlauf.

Die Länge und der radiale Wendeldurchmesser des wendelförmigen Teils der beiden länglichen Elemente sind dabei so gewählt, daß das distale Ende und ein an dieses angrenzender Bereich der beiden länglichen Elemente um die zwischen dem Stapediusmuskel und dem Steigbügel verlaufende Sehne gelegt und die wendelförmigen Teile entlang der Sehne geführt in Richtung auf den Stapediusmuskel bewegt und zumindest teilweise in den an die Sehne angrenzenden Bereich des Muskelbauchs eingedreht und/oder auf diesen Bereich aufgeschoben werden können. Die beiden länglichen Elemente sind zur Ermöglichung einer bipolaren Ableitung durch die Elektrode gegeneinander elektrisch isoliert, wobei aber entlang eines durch Aufschieben auf einen Muskelbauch mit diesem in Kontakt bringbaren wendelförmigen Abschnitts jedes länglichen Elements keine Isolation vorgesehen ist, die in diesem Zustand einen elektrischen Kontakt mit dem Muskelbauch verhindern könnte. Dabei ist es bevorzugt, wenn die beiden länglichen Elemente durch ein isolierendes Material aneinander befestigt sind. In jedem Fall sind die wendelförmigen Teile der beiden länglichen Elemente so ineinander verschachtelt angeordnet, daß sie eine gemeinsame Mittellinie umlaufen bzw. ein gemeinsames Volumen wenigstens teilweise umschließen und zusammen in vorteilhafter Weise als Einheit um die Sehne gelegt, entlang der Sehne geführt und mit dem Stapediusmuskel in Kontakt gebracht werden können. Dabei ist es insbesondere von Vorteil, wenn die wendelförmigen Teile so ausgebildet sind, daß sie durch Drehung um ihre gemeinsame Mittellinie (wobei sich die beiden länglichen Elemente insgesamt um die Mittellinie drehen) schraubenartig als Einheit entlang der Sehne geführt in Richtung auf den Stapediusmuskel bewegt und zumindest teilweise in den an die Sehne angrenzenden Bereich des Muskelbauchs eingedreht und/oder auf diesen Bereich aufgeschoben werden können.

Die ineinander verschachtelte Anordnung der beiden wendelförmigen Teile der beiden länglichen Elemente mit einer gemeinsamen Mittellinie bedeutet mit anderen Worten, daß die wendelförmigen Teile ineinander verschlungen sind und einander wendelförmig umlaufen. Dabei sind sie entlang der Mittellinie gegeneinander so versetzt, daß sich zwischen den beiden Windungen eines oder mehrerer Paare benachbarter Windungen des wendelförmigen Teils von einem der beiden länglichen Elemente jeweils mindestens eine Windung des wendelförmigen Teils des anderen länglichen Elements befindet, d.h. zumindest zwischen zwei Windungen eines länglichen Elements befindet sich mindestens eine Windungen des anderen länglichen Elements.

Das Legen der distalen Spitze und des an diese angrenzenden Bereichs jedes länglichen Elements um die Sehne kann dabei so erfolgen, daß die Sehne lediglich teilweise umschlossen wird, oder so, daß die Sehne vollständig umschlossen wird. Enthält der wendelförmige Teil eines länglichen Elements mindestens eine vollständige Windung, dann wird das längliche Element zunächst bevorzugt mit seiner abschließenden Windung vollständig um die Sehne gelegt, d.h. der an die distale Spitze angrenzende Bereich des länglichen Elements umfaßt in diesem Fall zumindest die abschließende Windung. Bei mehreren Windungen und der vorteilhaften Drehung um die Mittellinie wird dann beispielsweise eine Windung nach der anderen auf die Sehne "aufgefädelt". Enthält der wendelförmige Teil eines länglichen Elements weniger als eine vollständige Windung, dann wird das längliche Element so um die Sehne gelegt, daß es diese nur teilweise umschließt. In jedem Fall bewirkt die Sehne aufgrund des zumindest teilweise wendelförmigen Verlaufs der beiden länglichen Elemente, insbesondere bei der vorteilhaften schraubenartigen Bewegung der länglichen Elemente, eine sichere Führung der Elektrode in Richtung auf den Muskelbauch. Auf diese Weise kann die Elektrode ohne Spezialinstrumente in einfacher und definierter Weise entlang der Sehne "vorgeschraubt" und auf den Muskelbauch "auf- und/oder eingeschraubt" werden. Dabei dient die Sehne als Gleitschiene, an der entlang die wendelförmigen Teile in das Muskelgewebe eingedreht werden können.

Zur sichereren Befestigung kann das distale Ende von einem oder beiden länglichen Elementen auch einen oder mehrere Widerhaken aufweisen, wobei durch diese aber die Gefahr einer minimalen Traumatisierung besteht.

Die erfindungsgemäße Stapediusmuskelelektrodenanordnung kann in vorteilhafter Weise bei minimaler Traumatisierung eine sichere Befestigung und einen engen Kontakt der Elektrode mit dem Stapediusmuskelgewebe gewährleisten. In dieser Anordnung kann sie die bei einer Kontraktion des Stapediusmuskels erzeugten Aktionsströme aufnehmen und über eine geeignete Verbindung an eine Auswerteeinrichtung weiterleiten. Die elastischen Eigenschaften beiden länglichen Elemente sind so gewählt, daß die von ihnen auf den Muskelbauch ausgeübten Kräfte eine sichere Fixierung gegen Dislozierung und Rejektion infolge von Muskelbewegungen bewirken und gleichzeitig eine Drucknekrose oder sonstige Traumatisierung verhindert wird. Durch umgekehrte Drehung der wendelförmigen Abschnitte um ihre Mittellinie läßt sich die Elektrode darüber hinaus im wesentlichen atraumatisch wieder entfernen. Außerdem wird durch die wendelförmige Ausbildung eine große elektrische Kontaktfläche zwischen Elektrode und Gewebe erreicht.

In einer bevorzugten Ausführungsform ist das distale Ende von einem der beiden länglichen Elemente oder bevorzugt von beiden länglichen Elementen als Schneidspitze ausgebildet. Auf diese Weise ist eine einfache und im wesentlichen atraumatische Einführung des wendelförmigen Teils des länglichen Elements in das Muskelgewebe möglich.

In einer bevorzugten Ausführungsform sind die wendelförmigen Teile der beiden länglichen Element in der Weise ausgebildet, daß sie bei der schraubenartigen Bewegung in Richtung auf den Muskelbauch auf den an die Sehne angrenzenden Bereich des Muskelbauchs aufgeschoben werden können und sich dabei radial aufdehnen und federnd gegen den Muskelbauch drücken. Dies kann durch geeignete Wahl der elastischen Eigenschaften der beiden länglichen Elemente erreicht werden. Im befestigten Zustand verläuft dann ein vorderer Bereich des wendelförmigen Teils der beiden länglichen Elemente auf der Oberfläche des an die Sehne anschließenden Bereichs des Muskelbauchs und drückt federnd gegen diesen. Die elastischen Eigenschaften der beiden länglichen Elemente sind so gewählt, daß die von ihnen auf den Muskelbauch ausgeübten Kräfte eine sichere Fixierung gegen Dislozierung und Rejektion infolge von Muskelbewegungen bewirken und gleichzeitig eine Drucknekrose oder sonstige Traumatisierung verhindert wird. Es ist ferner besonders bevorzugt, wenn diese Ausführungsform mit der oben beschriebenen Ausbildung des distalen Endes von einem oder beiden länglichen Elementen als Schneidspitze kombiniert wird. Mit Hilfe der Schneidspitze bzw. der Schneidspitzen kann sich die Elektrode dann bei dem Aufschieben des wendelförmigen Teils der beiden länglichen Elemente auf den Muskelbauch teilweise in dessen Oberfläche einschneiden, um in ihr eine Vertiefung auszubilden, die die Fixierung verbessert. Da lediglich die Oberfläche des Muskelbauchs betroffen ist, ist mit der Verwendung der Schneidspitze keine praktisch relevante Traumatisierung verbunden.

In einer vorteilhaften Ausgestaltung sind die wendelförmigen Teile der beiden länglichen Elemente so ineinander verschachtelt angeordnet, daß zwischen jeden zwei benachbarten Windungen des wendelförmigen Teils von einem der beiden länglichen Elemente mindestens eine Windung des wendelförmigen Teils des anderen länglichen Elements angeordnet ist. Dabei können die beiden wendelförmigen Teile dieselbe oder eine unterschiedliche Steigung bzw. Windungszahl pro Längeneinheit aufweisen. Im letztere Fall befindet sich nicht zwischen jeden zwei benachbarten Windungen des anderen länglichen Elements mindestens eine Windung des ersteren länglichen Elements. Es ist jedoch bevorzugt, daß die wendelförmigen Teile der beiden länglichen Elemente so ineinander verschachtelt angeordnet sind, daß die Steigung gleich oder ungefähr gleich ist und zwischen jeden zwei benachbarten Windungen des wendelförmigen Teils von jedem der beiden länglichen Elemente genau eine Windung des wendelförmigen Teils des anderen länglichen Elements angeordnet ist, so daß die beiden wendelförmigen Teile in Form einer Doppelhelix angeordnet sind. Mit anderen Worten stellen die beiden wendelförmigen Teile zwei umeinanderlaufende einfache Helices dar und zeigen einen Verlauf, der mit dem einer zweigängigen Schraube vergleichbar ist. Die einzelnen Windungen der beiden wendelförmigen Teile wechseln sich entlang der Mittellinie alternierend ab.

Es ist bevorzugt, daß die beiden länglichen Elemente oder zumindest die wendelförmigen Teile der beiden länglichen Elemente eine identische Form und identische Abmessungen oder eine im wesentlichen identische Form und im wesentlichen identische Abmessungen haben.

In einer bevorzugten Ausführungsform sind die distalen Enden der beiden länglichen Elemente unmittelbar benachbart zueinander angeordnet und bilden zusammen das distale Ende der Elektrode. Dabei wird im allgemeinen das distale Ende eines der beiden länglichen Elemente entlang der Mittellinie in einem geringen Abstand hinter dem distalen Ende des anderen länglichen Elements angeordnet sein. Wenn die beiden länglichen Elemente oder ihre wendelförmigen Teile eine identische Form und identische Abmessungen haben, so folgt jeder wendelförmige Teil versetzt dem Verlauf des anderen wendelförmigen Teils und der Verlauf des durch die beiden wendelförmigen Teile gebildeten Teils der Elektrode entspricht dem Verlauf der beiden wendelförmigen Teile. Im Fall identischer länglicher Elemente hat die Elektrode denselben Verlauf und dieselbe Länge wie die beiden länglichen Elemente.

Es ist bevorzugt, daß die wendelförmigen Teile der beiden länglichen Elemente an jeder Position entlang der gemeinsamen Mittellinie denselben Wendeldurchmesser haben. Dieser Durchmesser kann entlang der Mittellinie konstant sein oder sich entlang der Mittellinie abschnittsweise oder kontinuierlich ändern. Lokal stimmt der Durchmesser jedoch an jedem Punkt entlang der Mittellinie überein. Auf diese Weise wird in vorteilhafter Weise sichergestellt, daß keiner der wendelförmigen Teile der beiden länglichen Elemente in das von dem wendelförmigen Teil des anderen länglichen Elements umschlossene Volumen hineinragt bzw. vorsteht. Vielmehr umschließen die beiden wendelförmigen Teile ein gemeinsames Volumen mit "glatter" Oberfläche.

Es ist bevorzugt, daß die wendelförmigen Teile der beiden länglichen Elemente entlang der gemeinsamen Mittellinie um weniger als eine Windungslänge von mindestens einem der beiden wendelförmigen Teile gegeneinander versetzt sind.

Es ist bevorzugt, daß die beiden länglichen Elemente einen Durchmesser von 20 bis 300 um und mehr bevorzugt von 50 bis 180 µm aufweisen. Durch die Wahl eines solchen Durchmessers lassen sich eine sichere Befestigung mit einer fehlenden oder höchstens minimalen Traumatisierung verbinden. Bei einem nicht kreisförmigen Querschnitt bezeichnet der oben angegebene Bereich den Bereich, in dem sowohl der maximale als auch der minimale Durchmesser liegen. Die beiden länglichen Elemente können gleiche oder verschiedene Durchmesser aufweisen.

Der Querschnitt der beiden länglichen Elemente oder zumindest ihres wendelförmigen Teils kann kreisförmig, halbkreisförmig, oval, quadratisch, quadratisch mit abgerundeten Ecken, rechteckig, rechteckig mit abgerundeten Ecken oder ringabschnittsförmig sein. Durch eine geeignete Querschnittswahl lassen sich gezielt die mechanischen Eigenschaften der länglichen Elemente beeinflussen und z.B. unterschiedliche axiale Biegesteifigkeiten gegenüber Biegungen in verschiedene Richtungen erreichen. Der Querschnitt ist für die beiden länglichen Elemente bevorzugt identisch, kann jedoch auch unterschiedlich sein.

Es ist bevorzugt, daß die beiden länglichen Elemente eine axiale Biegesteifigkeit von 5 bis 60 N mm², mehr bevorzugt von 5 bis 50 N mm² und am meisten bevorzugt von etwa 30 N mm²aufweist. Diese Werte können für die beiden länglichen Elemente identisch oder verschieden sein. Dabei kann es vorteilhaft sein, wenn mindestens eines der beiden länglichen Elemente so ausgestaltet ist, daß die axiale Biegesteifigkeit von dem proximalen Ende dieses länglichen Elements zu dem distalen Ende dieses länglichen Elements abnimmt, z.B. von 60 N mm² am proximalen Ende auf 25 N mm² am distalen Ende. Dadurch übt das entsprechende wendelförmige Teil bei Erreichen des Muskelbauchs zunächst keine starken Kräfte auf diesen aus und kann z.B. einfach aufgedehnt und auf den Muskelbauch aufgeschoben werden. Im weiteren Verlauf des Befestigungsvorgangs erreichen dann Abschnitte des wendelförmigen Teils des jeweiligen länglichen Elements den Muskelbauch, die größere Kräfte ausüben und für eine sichere Befestigung sorgen. Diese Abschnitte werden durch die bereits mit dem Muskelbauch in Verbindung stehenden, distaleren Abschnitte geführt.

Es ist ferner bevorzugt, daß die wendelförmigen Teile der beiden länglichen Elemente einen Querschnittsdurchmesser von 80 bis 800 µm und mehr bevorzugt von 100 bis 500 µm aufweisen. Dieser Querschnittsdurchmesser bezieht sich auf den Durchmesser einer Querschnittsfläche des von dem wendelförmigen Abschnitt umschlossenen bzw. definierten Volumens senkrecht zur Erstreckungsrichtung dieses Volumens bzw. des wendelförmigen Teils. Bei einem nicht kreisförmigen Querschnitt bezeichnet der oben angegebene Bereich den Bereich, in dem sowohl der maximale als auch der minimale Durchmesser liegen. Der Querschnittsdurchmesser kann für die beiden länglichen Elemente identisch oder unterschiedlich sein.

Es ist bevorzugt, daß die wendelförmigen Teile der beiden länglichen Elemente in ihrer Erstreckungsrichtung eine Länge von 0,1 bis 3 mm und mehr bevorzugt von 0,5 bis 1,5 mm aufweisen. Diese Länge kann für die beiden länglichen Elemente identisch oder auch unterschiedlich sein.

Ferner ist es bevorzugt, daß die Anzahl von Windungen der wendelförmigen Teile der beiden längliche Elementel ¼ bis sechs und mehr bevorzugt ¼ bis 1 beträgt. Die Anzahl von Windungen gibt in üblicher Weise an, wie oft der wendelförmige Teil seine Mittellinie vollständig umläuft. Eine geringe Windungszahl hat den Vorteil, daß das "Auffädeln" auf die Sehne nur wenige Umdrehungen der Elektrodenanordnung erfordert, so daß Schwierigkeiten mit einer Verdrehung der Ableitungen vermieden werden. Die beiden länglichen Elemente können identische oder auch unterschiedliche Windungszahlen haben.

Es ist bevorzugt, daß die beiden länglichen Elemente Edelstahl, insbesondere chirurgischen Edelstahl 316L (X5CrNiMo18.10), eine CrCoMo-Legierung, eine NiTi-Legierung, insbesondere eine NiTi-Memory-Legierung, Platin, eine PtIr-Legierung, Silber, Gold, Palladium, Tantal oder Titan oder eine Legierung von diesen aufweisen. Die Materialzusammensetzung der beiden länglichen Elemente ist bevorzugt identisch, kann aber auch unterschiedlich sein.

Ferner ist es bevorzugt, daß die beiden länglichen Elemente oder eines von ihnen zumindest teilweise mit Siliziumkarbid, pyrolytischem Kohlenstoff und/oder diamantartigem Kohlenstoff beschichtet ist. Durch eine solche Beschichtung kann die wirksame Elektrodenfläche erhöht werden. Dabei ist insbesondere eine fraktale Beschichtung von Vorteil. Eine Erhöhung der wirksamen Oberfläche läßt sich alternativ oder zusätzlich auch durch die Ausbildung von axialen Längsrillen in einem oder beiden länglichen Elementen erreichen. Eine solche Ausgestaltung hat auch den Vorteil einer sichereren Befestigung der Elektrode.

Es kann auch von Vorteil sein, wenn die beiden länglichen Elemente oder zumindest eines von ihnen entlang eines Teils ihrer Länge mit einer Isolation versehen ist, die bevorzugt aus einem hydrolysestabilisierten Silikonelastomer, Polyether oder Polyetherurethanelastomer besteht. Diese Isolation kann z.B. in einem nicht wendelförmigen Teil der länglichen Elemente und/oder in einem proximalen Bereich der wendelförmigen Teile vorgesehen sein. In dem Fall einer Isolation bildet der nicht isolierte Bereich der länglichen Elemente die eigentliche Elektrode. Dieser nicht isolierte Bereich muß dementsprechend wie oben angegeben selbst zumindest entlang eines Teils seiner Länge wendelförmig sein.

In einer bevorzugten Ausführungsform weist jedes der beiden länglichen Elemente einen Draht auf oder ist bevorzugt ein Draht.

Es ist ferner bevorzugt, daß jedes der beiden länglichen Elemente mit einer elektrischen Leitung zur Ableitung der von der Elektrode aufgenommenen Aktionsströme verbunden ist. Diese Leitungen sind bevorzugt dünner als das jeweilige längliche Element und insgesamt so dünn wie möglich ausgebildet, um der Handhabung der Elektrodenanordnung bei der Befestigung am Stapediusmuskel möglichst wenig Widerstand entgegenzusetzen.

Die Elektrode kann nicht nur mit zwei länglichen Elementen als bipolare Elektrode für bipolare Ableitungen ausgebildet sein. Es ist auch möglich, daß noch mehr längliche Elemente vorgesehen sind, um eine multipolare Elektrode zu bilden.

Die oben beschriebene Stapediusmuskelelektrodenanordnung ist in einer bevorzugten Ausführungsform Teil eines Systems, das neben der Stapediusmuskelelektrodenanordnung ein längliches Einführinstrument zur Einführung der Stapediusmuskelelektrodenanordnung in einen menschlichen Körper und zur Befestigung der Stapediusmuskelelektrode an einem menschlichen Stapediusmuskel aufweist. Das proximale Ende von mindestens einem der beiden länglichen Elemente (oder bei multipolaren Elektroden ggf. des bzw. der weiteren länglichen Elemente) ist lösbar mit einer Spitze des Einführinstruments verbunden. Die Verbindung ist so ausgestaltet, daß das Einführinstrument nach Befestigung der Elektrode am Stapediusmuskel durch Zug und/oder Drehung des Einführinstruments von der Elektrodenanordnung gelöst werden kann. In einer besonders bevorzugten Ausführungsform ist die mechanische Verbindung zwischen der Spitze des Einführinstruments und dem oder den proximalen Enden der länglichen Elemente mit einer Schwächungszone versehen, die eine Sollbruchstelle bereitstellt. Dadurch kann das Einführinstrument bei atraumatischem Kraftaufwand in definierter Weise von der Elektrodenanordnung getrennt werden.

Die Erfindung wird im folgenden anhand von Ausführungsbeispielen unter Bezugnahme auf die Zeichnungen näher erläutert.
- Figur 1: zeigt eine schematische Ansicht einer erfindungsgemä- ßen Stapediusmuskelelektrodenanordnung.
- Figur 2: zeigt eine schematische Schnittansicht eines Teils des Mittelohrs mit einer erfindungsgemäßen Stapedius- muskelelektrodenanordnung, deren Elektrode an dem Stapediusmuskel befestigt wird.

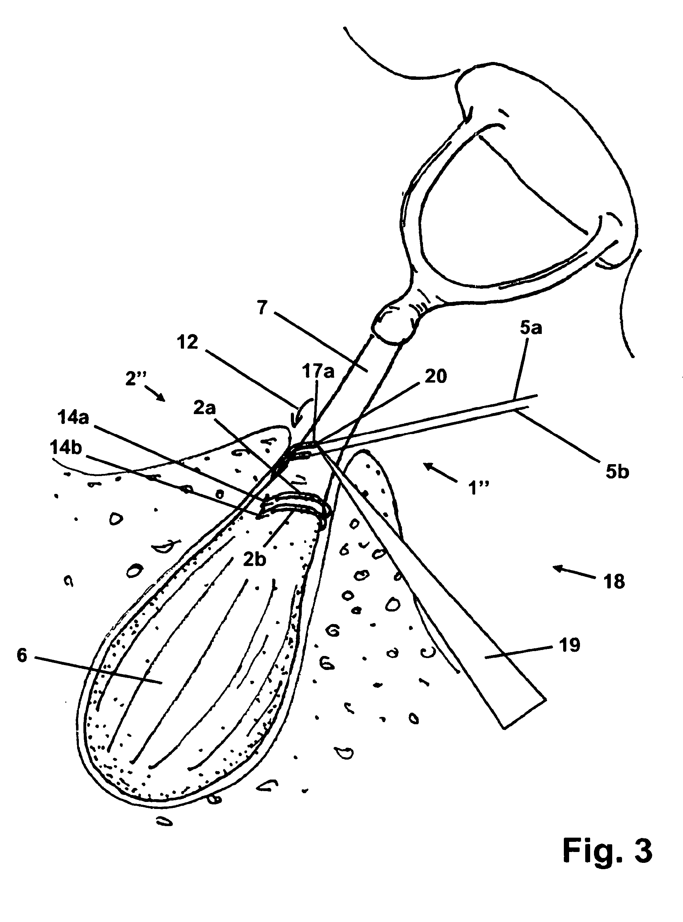

- Figur 3: zeigt eine schematische Schnittansicht eines Teils des Mittelohrs mit einer erfindungsgemäßen, mit einem Einführinstrument verbundenen Stapediusmuskelelektro- denanordnung, deren Elektrode an dem Stapediusmuskel befestigt wird.

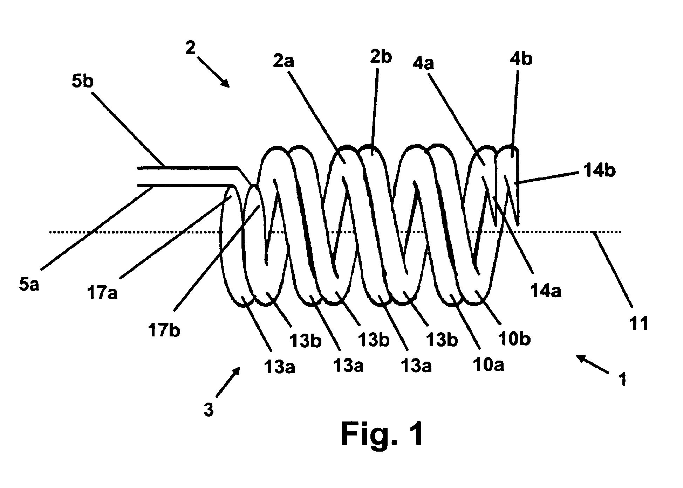

Die in Figur 1 gezeigte Stapediusmuskelelektrodenanordnung 1 enthält eine bipolare Elektrode 2, die einen ersten flexiblen, elastischen Draht 2a aus einem elektrisch leitenden und biokompatiblen Material und einen zweiten flexiblen, elastischen Draht 2b aus einem elektrisch leitenden und biokompatiblen Material aufweist. Jeder der beiden Drähte 2a, 2b ist entlang seiner gesamten Länge wendelförmig vorgeformt, und die beiden Drähte 2a, 2b haben eine identische Form und identische Abmessungen. Sie sind in der Weise ineinander verschachtelt, daß sie eine gemeinsame Mittellinie 11 umlaufen und ein gemeinsames Volumen umschließen, und verlaufen um einen Bruchteil einer Windungslänge entlang der Mittellinie 11 gegeneinander versetzt, so daß sie zusammen eine Doppelhelix bilden. Der Draht 2b ist entlang seiner gesamten Länge an der in Figur 1 rechten Seite des Drahtes 2a befestigt, wobei die beiden Drähte 2a, 2b durch eine nicht gezeigte Isolierung elektrisch gegeneinander isoliert sind. Die Befestigung der beiden Drähte 2a, 2b aneinander wird bevorzugt durch ein geeignetes isolierendes Material bewirkt, wie z.B. durch eine zwischen den beiden Drähten 2a, 2b vorgesehene Kunststoffschicht. Abgesehen von der Isolierung zwischen den Drähten 2a, 2b sind die Drähte 2a, 2b unisoliert, so daß sie entlang ihrer gesamten Länge mit einem Stapediusmuskel in elektrischen Kontakt gebracht werden können. Aus Figur 1 ist ersichtlich, daß die beiden Drähte 2a, 2b zusammen als ein einziges, wendelförmig vorgeformtes längliches Element angesehen werden können, dessen allgemeiner Verlauf dem Verlauf der beiden Drähte 2a, 2b entspricht. Daher ist die Elektrode 2 selbst ein wendelförmiges längliches Element, dessen Verlauf dem Verlauf der beiden einzelnen Drähte 2a, 2b entspricht.

Jeder Draht 2a, 2b ist an seinem proximalen Ende 17a bzw. 17b mit einer elektrischen Leitung 5a bzw. 5b verbunden, die als elektrische Verbindung zwischen dem jeweiligen Draht 2a bzw. 2b und einer geeigneten Auswerteeinrichtung (nicht gezeigt) dient. Alternativ könnten statt separaten Leitungen 5a, 5b auch Drähte 2a, 2b vorgesehen sein, die einen distalen Abschnitt bzw. Teil 3a, 3b, in denen der jeweilige Draht 2a bzw. 2b wendelförmig vorgeformt ist und der sich bis zu dem distalen Ende 4a bzw. 4b des Drahtes 2a bzw. 2b erstreckt, und einen proximalen, nicht wendelförmigen Abschnitt 5a bzw. 5b aufweisen, der als die elektrische Verbindung zwischen dem wendelförmigen Abschnitt 3a bzw. 3b und einer geeigneten Auswerteeinrichtung (nicht gezeigt) dient. Es sind auch Mischformen möglich, d.h. ein Draht könnte vollständig wendelförmig und mit einer separaten Leitung verbunden sein, während der andere Draht einen als elektrische Verbindung verwendbaren proximalen, nicht wendelförmigen Abschnitt aufweist, oder einer oder beide Drähte könnten einen nicht wendelförmigen proximalen Abschnitt aufweisen, der an seinem proximalen Ende mit einer separaten elektrischen Leitung verbunden ist.

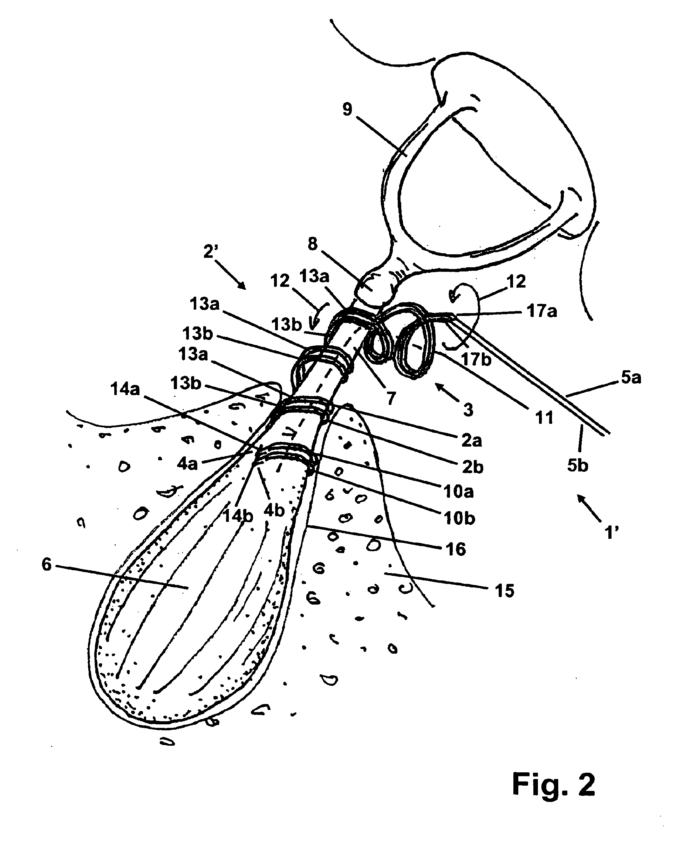

An seinem distalen Ende 4a, 4b ist jeder Draht 2a, 2b mit einer Schneidspitze 14a, 14b versehen, mit deren Hilfe sich die Elektrode 2, wie nachfolgend unter Bezugnahme auf die Figur 2 erläutert wird, in die Oberfläche eines Stapediusmuskels einschneiden kann, um den Halt der Elektrode 2 an dem Stapediusmuskel zu verbessern.

Aus Figur 1 ist ersichtlich, daß jeder Draht 2a, 2b und damit die gesamte doppelhelixförmige Elektrode 2 vier Windungen aufweist. In Figur 1 ist die vorderste Windung mit den Bezugszeichen 10a, 10b gekennzeichnet, während die restlichen Windungen mit den Bezugszeichen 13a, 13b gekennzeichnet sind.

In Figur 2 ist eine Stapediusmuskelelektrodenanordnung 1' während des Vorgangs der Befestigung der Elektrode 2' an dem Bauch 6 eines Stapediusmuskels gezeigt. Die Stapediusmuskelelektrodenanordnung 1' ist mit der in Figur 1 gezeigten Stapediusmuskelelektrodenanordnung 1 bis auf den Unterschied identisch, daß ihre Elektrode 2' fünf Windungen 10a, 10b, 13a, 13b aufweist, während die Elektrode 2 aus Figur 1 nur vier Windungen 10a, 10b, 13a, 13b hat. Aus Gründen der Übersichtlichkeit sind die beiden Drähte 2a, 2b der Elektrode 2' in einem gewissen Abstand zueinander ohne eine erkennbare Befestigungseinrichtung zwischen den Drähten 2a, 2b dargestellt. Selbstverständlich sind die Drähte 2a, 2b aber entweder wie bei der Ausführungsform der Figur 1 entlang ihrer gesamten Länge durch eine zwischen ihnen angeordnete isolierende Materialschicht oder durch einzelne, voneinander beabstandete Befestigungseinrichtungen miteinander verbunden.

Wie aus Figur 2 ersichtlich ist, ist der Bauch 6 des Stapediusmuskels über eine Sehne 7 mit dem Kopf 8 des Steigbügels 9 verbunden. Zur Implantation der Stapediusmuskelelektrode 2' wird zunächst ihre vorderste Windung 10a, 10b um die Sehne 7 herumgelegt (bzw. die beiden Drähte 2a, 2b werden mit ihrer vordersten Windungen 10a bzw. 10b gleichzeitig um die Sehne 7 herumgelegt). Anschließend werden die Drähte 2a, 2b (zusammen mit den Leitungen 5a, 5b) bzw. die Elektrode 2' als Einheit um die Mittellinie 11 gedreht, wie es durch die Pfeile 12 angegeben ist. Durch die wendelförmige Ausbildung der Drähte 2a, 2b und daher der Elektrode 2' wird diese dadurch schraubenartig entlang der Sehne 7 in Richtung auf den Muskelbauch 6 vorgeschoben, wobei mit jeder Umdrehung eine weitere Windung 13a, 13b der Drähte 2a, 2b und damit der Elektrode 2' auf die Sehne 7 "aufgefädelt" und die Elektrode 2' um einen Windungsabstand entlang der Sehne in Richtung auf den Stapediusmuskel bewegt wird. Wie der Figur 2 zu entnehmen ist, ist der radiale Querschnittsdurchmesser der Windungen 10a, 10b, 13a, 13b so gewählt, daß die Windungen 10a, 10b, 13a, 13b die Sehne 7 mit etwas Spiel umgeben.

Nach einiger Zeit erreicht das vordere Ende 4a, 4b der Elektrode 2' den Muskelbauch 6. Die Drehung wird dann fortgesetzt, um eine Anzahl der Windungen 13a, 13b (von denen in Figur 2 nur drei durch Bezugszeichen gekennzeichnet sind) auf den an die Sehne 7 angrenzenden Bereich des Muskelbauchs 6 aufzuschieben bzw. gewissermaßen "aufzuschrauben". Dabei ist der radiale Durchmesser der wendelförmigen Drähte 2a, 2b so gewählt, daß sich diese bei dem Aufschieben radial aufweiten müssen. Aufgrund der elastischen Ausbildung, drücken die Drähte 2a, 2b und damit die Elektrode 2' gegen die Oberfläche des Muskelbauchs 6 und werden dadurch auf dieser fixiert. Durch umgekehrte Drehung der Stapediusmuskelelektrode 2' kann sie von dem Muskelbauch 6 und von der Sehne 7 gelöst und entfernt werden. Die Fixierung wird zusätzlich durch die an den distalen Enden 4a, 4b der Drähte 2a, 2b vorgesehenen Schneidspitzen 14a, 14b verbessert, die jeweils eine flache Vertiefung (nicht dargestellt) in die Oberfläche des Muskelbauchs 6 schneiden, in der der jeweilige Draht 2a bzw. 2b verläuft.

Die Stapediusmuskelelektrode 2, 2' kann in einfacher Weise an dem in einem im Knochen 15 ausgebildeten Kanal 16 befestigt werden, ohne daß die knöcherne Struktur 15 verändert werden muß. Dabei tritt auch bei Einsatz der Schneidspitzen 14a, 14b keine praktisch relevante Traumatisierung des Muskelbauchs 6 auf.

In Figur 2 ist eine weitere Stapediusmuskelelektrodenanordnung 1 " gezeigt, die sich von den in den Figuren 1 und 2 gezeigten Stapediusmuskelelektrodenanordnungen 1 und 1' zunächst dadurch unterscheidet, daß ihre Elektrode 2" bzw. die beiden Drähte 2a, 2b der Elektrode 2" nur eine vollständige Windung 10a, 10b und damit gewissermaßen nur eine abschließende Windung aufweisen. Es ist darauf hinzuweisen, daß die Elektrode 2" auch so ausgebildet sein kann, daß sie und ihre Drähte 2a, 2b weniger als eine vollständige Windung 10a, 10b aufweisen. Ferner ist die Stapediusmuskelelektrodenanordnung 1" Teil eines Systems 18, das neben der Stapediusmuskelelektrodenanordnung 1" noch ein längliches Einführinstrument 19 aufweist. Dieses ist mit einer Spitze mechanisch mit dem proximalen Ende 17a des Drahtes 2a verbunden. Diese Verbindung ist mit einer zu einer Sollbruchstelle führenden Schwächungszone 20 versehen, so daß das Einführinstrument 19 nach Befestigung der Elektrode 2 " an dem Muskelbauch 6 in einfacher Weise und ohne die Gefahr einer Traumatisierung des Muskelbauchs 6 oder einem Lösen der Elektrode 2 " von dem Muskelbauch 6 durch Zug und/oder Drehung in definierter Weise von der Elektrodenanordnung 1 " abgesprengt werden kann. Es ist auch möglich, daß das längliche Einführinstrument 19 nicht nur mit dem proximalen Ende von einem der beiden Drähte 2a, 2b verbunden ist, sondern mit den proximalen Enden 17a, 17b von beiden Drähten 2a, 2b.

## Patentansprüche

1. Stapediusmuskelelektrodenanordnung zur Detektion der bei einer Kontraktion eines menschlichen Stapediusmuskels erzeugten Aktionsströme, die eine für eine Ableitung angepaßte Elektrode (2, 2', 2") zur Befestigung an einem menschlichen Stapediusmuskel umfaßt, die zwei flexible, elastische, elektrisch leitende längliche Elemente (2a, 2b) aufweist, von denen jedes ein distales (4a, 4b) und ein proximales Ende (17a, 17b) hat und entlang zumindest eines Teils seiner Länge bis zu seinem distalen Ende (4a, 4b) in der Weise wendelförmig vorgeformt ist, daß das distale Ende (4a, 4b) und ein an dieses angrenzender Abschnitt des jeweiligen länglichen Elements (2a, 2b) zumindest teilweise um die zwischen dem Stapediusmuskel und dem Steigbügel verlaufende Sehne (7) gelegt und der wendelförmige Teil entlang der Sehne (7) geführt in Richtung auf den Stapediusmuskel- bewegt und zumindest teilweise in den an die Sehne (7) angrenzenden Bereich des Muskelbauchs (6) eingedreht und/oder auf diesen Bereich aufgeschoben werden kann, und ihre wendelförmigen Teile (3) so ineinander verschachtelt angeordnet sind, daß sie eine gemeinsame Mittellinie (11) umlaufen und zusammen um die Sehne (7) gelegt, entlang der Sehne (7) geführt und mit dem Stapediusmuskel in Kontakt gebracht werden können, **dadurch gekennzeichnet, dass** die Elektrode für eine bipolare Ableitung angepasst ist, wobei die beiden länglichen Elemente (2a, 2b) gegeneinander elektrisch isoliert sind.

2. Stapediusmuskelelektrodenanordnung nach Anspruch 1, bei der das distale Ende (4a, 4b) von mindestens einem der beiden länglichen Element (2a, 2b) als Schneidspitze (14a, 14b) ausgebildet ist.

3. Stapediusmuskelelektrodenanordnung nach Anspruch 1 oder Anspruch 2, bei der die wendelförmigen Teile (3) der beiden länglichen Elemente (2a, 2b) in der Weise ausgebildet sind, daß sie auf den an die Sehne (7) angrenzenden Bereich des Muskelbauchs (6) aufgeschoben werden können und sich dabei radial aufdehnen und federnd gegen den Muskelbauch (6) drücken.

4. Stapediusmuskelelektrodenanordnung nach einem der vorhergehenden Ansprüche, bei der die wendelförmigen Teile (3) der beiden länglichen Elemente (2a, 2b) so ineinander verschachtelt angeordnet sind, daß zwischen jeden zwei benachbarten Windungen (10a, 13a) einem der beiden länglichen Elemente (2a) mindestens eine Windung (10b, 13b) des anderen länglichen Elements (2b) angeordnet ist,
wobei vorzugsweise die wendelförmigen Teile (3) der beiden länglichen Elemente (2a, 2b) so ineinander verschachtelt angeordnet sind, daß zwischen jeden zwei benachbarten Windungen (10a, 13a) von einem der beiden länglichen Elemente (2a) genau eine Windung (10b, 13b) des anderen länglichen Elements (2b) angeordnet ist, so daß die beiden wendelförmigen Teile (3) in Form einer Doppelhelix angeordnet sind.

5. Stapediusmuskelelektrodenanordnung nach einem der vorhergehenden Ansprüche, bei der die beiden länglichen Elemente (2a, 2b) eine identische Form und identische Abmessungen haben, und/oder
bei der die distalen Enden (4a, 4b) der beiden länglichen Elemente (2a, 2b) unmittelbar benachbart zueinander angeordnet sind und zusammen das distale Ende der Elektrode (2, 2', 2") bilden.

6. Stapediusmuskelelektrodenanordnung nach einem der vorhergehenden Ansprüche, bei der die wendelförmigen Teile (3) der beiden länglichen Elemente (2a, 2b) an jeder Position entlang der gemeinsamen Mittellinie (11) denselben Wendeldurchmesser haben, und/oder bei der die wendelförmigen Teile (3) der beiden länglichen Elemente (2a, 2b) entlang der gemeinsamen Mittellinie (11) um weniger als einen Windungsabstand von mindestens einem der beiden wendelförmigen Teile (3) gegeneinander versetzt sind.

7. Stapediusmuskelelektrodenanordnung nach einem der vorhergehenden Ansprüche, bei der die beiden länglichen Elemente (2a, 2b) einen Durchmesser von 20 bis 300 um aufweisen, insbesondere
einen Durchmesser von 50 bis 180 µm aufweisen.

8. Stapediusmuskelelektrodenanordnung nach einem der vorhergehenden Ansprüche, bei der der Querschnitt der beiden länglichen Elemente (2a, 2b) kreisförmig, halbkreisförmig, oval, quadratisch, quadratisch mit abgerundeten Ecken, rechteckig oder rechteckig mit abgerundeten Ecken ist, und/oder
bei der die beiden länglichen Elemente (2a, 2b) eine axiale Biegesteifigkeit von 5 bis 60 N mm² aufweisen, wobei
mindestens eines der beiden länglichen Elemente (2a, 2b) vorzugsweise so ausgestaltet ist, daß die axiale Biegesteifigkeit von dem proximalen Ende (17a, 17b) des länglichen Elements (2a, 2b) zu dem distalen Ende (4a, 4b) des länglichen Elements (2a, 2b) abnimmt.

9. Stapediusmuskelelektrodenanordnung nach einem der vorhergehenden Ansprüche, bei der die wendelförmigen Teile (3) der beiden länglichen Elemente (2a, 2b) einen Querschnittsdurchmesser von 80 bis 800 µm aufweisen, und/oder
bei der die wendelförmigen Teile (3) der beiden länglichen Elemente (2a, 2b) in ihrer Erstreckungsrichtung eine Länge von 0,1 bis 3 mm aufweisen, und/oder
bei der die Anzahl von Windungen der wendelförmigen Teile (3) der beiden länglichen Elemente (2a, 2b) ¼ bis sechs beträgt.

10. Stapediusmuskelelektrodenanordnung nach einem der vorhergehenden Ansprüche, bei der die beiden länglichen Elemente (2a, 2b) Edelstahl, eine CrCoMo-Legierung, eine NiTi-Legierung, Platin, eine PtIr-Legierung, Silber, Gold, Palladium, Tantal oder Titan oder eine Legierung von diesen aufweisen, und/oder
bei der die beiden länglichen Elemente (2a, 2b) zumindest teilweise mit Siliziumkarbid, pyrolytischem Kohlenstoff und/oder diamantartigem Kohlenstoff beschichtet sind, wobei die Beschichtung vorzugsweise fraktal ausgebildet ist.

11. Stapediusmuskelelektrodenanordnung nach einem der vorhergehenden Ansprüche, bei der die beiden länglichen Elemente (2a, 2b) zumindest teilweise mit einer Polyurethan- oder Silikonelastomer-Isolation versehen sind.

12. Stapediusmuskelelektrodenanordnung nach einem der vorhergehenden Ansprüche, bei der die beiden länglichen Elemente (2a, 2b) jeweils durch einen Draht gebildet werden.

13. Stapediusmuskelelektrodenanordnung nach einem der vorhergehenden Ansprüche, bei der jedes der beiden länglichen Elemente (2a, 2b) mit einer elektrischen Leitung (5a, 5b) zur Ableitung der Aktionsströme verbunden ist.

14. System aus einer Stapediusmuskelelektrodenanordnung nach einem der Ansprüche 1 bis 13 und einem länglichen Einführinstrument (18) zur Einführung der Stapediusmuskelelektrodenanordnung (1") in einen menschlichen Körper und zur Befestigung der Stapediusmuskelelektrode (2") an einem menschlichen Stapediusmuskel, wobei das proximale Ende (17a, 17b) von mindestens einem der beiden länglichen Elemente (2a, 2b) lösbar mit einer Spitze des Einführinstruments (18) verbunden ist.

15. System nach Anspruch 14, bei dem die Verbindung zwischen der Spitze des Einführinstruments (18) und den proximalen Enden (17a, 17b) der beiden länglichen Elemente (2a, 2b) eine Schwächungszone (20) aufweist, die eine Sollbruchstelle bereitstellt.

## Claims

1. A stapedius muscle electrode configuration for the detection of the action currents generated upon a contraction of a human stapedius muscle, which comprises an electrode (2, 2', 2"), adapted for carrying a current and to be fastened on a human stapedius muscle, which has two flexible, elastic, electrically conductive oblong elements (2a, 2b), each one of which having a distal end (4a, 4b) and a proximal end (17a, 17b) and being pre-shaped coiled along at least a part of its length up to its distal end (4a, 4b) in such a way that the distal end (4a, 4b) and a section of the respective oblong element (2a, 2b) adjoining this end is laid at least partially around the tendon (7) running between the stapedius muscle and the stirrup and the coiled part may be moved along the tendon (7) while guided in the direction toward the stapedius muscle and at least partially screwed into the area of the muscle belly (6) adjoining the tendon (7) and/or pushed onto this area, wherein coiled parts (3) of the oblong elements are situated interleaved in one another so that they circle a shared center line (11) and may be laid around the tendon (7), guided along the tendon (7), and brought into contact with the stapedius muscle together, **characterized in that** the electrode is adapted for a bipolar current carrying and the two oblong elements (2a, 2b) are electrically insulated to one another.

2. The stapedius muscle electrode configuration according to Claim 1, wherein the distal end (4a, 4b) of at least one of the two oblong elements (2a, 2b) is implemented as a cutting tip (14a, 14b).

3. The stapedius muscle electrode configuration according to Claim 1 or Claim 2, wherein the coiled parts (3) of the two oblong elements (2a, 2b) are implemented in such a way that they may be pushed onto the area of the muscle belly (6) adjoining the tendon (7), and they expand radially and press elastically against the muscle belly (6).

4. The stapedius muscle electrode configuration according to one of the preceding claims, wherein the coiled parts (3) of the two oblong elements (2a, 2b) are situated interleaved in one another so that at least one turn (10b, 13b) of the other oblong element (2b) is situated between each two adjacent turns (10a, 13a) of one of the two oblong elements (2a), wherein preferably the coiled parts (3) of the two oblong elements (2a, 2b) are situated interleaved in one another so that precisely one turn (10b, 13b) of the other oblong element (2b) is situated between each two adjacent turns (10a, 13a) of one of the two oblong elements (2a), so that the two coiled parts (3) are situated in the form of a double helix.

5. The stapedius muscle electrode configuration according to one of the preceding claims, wherein the two oblong elements (2a, 2b) have an identical shape and identical dimensions, and/or
the distal ends (4a, 4b) of the two oblong elements (2a, 2b) are situated directly adjacent to one another and together form the distal end of the electrode (2, 2', 2").

6. The stapedius muscle electrode configuration according to one of the preceding claims, wherein the coiled parts (3) of the two oblong elements (2a, 2b) have the same coil diameter at every position along the shared center line (11), and/or
the coiled parts (3) of the two oblong elements (2a, 2b) are offset to one another along the shared center line (11) by less than one turn distance of at least one of the two coiled parts (3).

7. The stapedius muscle electrode configuration according to one of the preceding claims, wherein the two oblong elements (2a, 2b) have a diameter of 20 to 300 µm, in particular a diameter of 50 to 180 µm.

8. The stapedius muscle electrode configuration according to one of the preceding claims, wherein the cross-section of the two oblong elements (2a, 2b) is circular, semicircular, oval, square, square having rounded corners, rectangular, or rectangular having rounded corners, and/or
wherein the two oblong elements (2a, 2b) have an axial bending rigidity of 5 to 60 N mm², wherein at least one of the two oblong elements (2a, 2b) is preferably designed so that the axial bending rigidity decreases from the proximal end (17a, 17b) of the oblong element (2a, 2b) to the distal end (4a, 4b) of the oblong element (2a, 2b).

9. The stapedius muscle electrode configuration according to one of the preceding claims, wherein the coiled parts (3) of the two oblong elements (2a, 2b) have a cross-sectional diameter of 80 to 800 µm, and/or
wherein the coiled parts (3) of the two oblong elements (2a, 2b) have a length of 0.1 to 3 mm in their extension direction, and/or
wherein the number of turns of the coiled parts (3) of the two oblong elements (2a, 2b) is 1/4 to six.

10. The stapedius muscle electrode configuration according to one of the preceding claims, wherein the two oblong elements (2a, 2b) have stainless steel, a CrCoMo alloy, a NiTi alloy, platinum, a PtIr alloy, silver, gold, palladium, tantalum, or titanium or an alloy thereof, and/or wherein the two oblong elements (2a, 2b) are at least partially coated with silicon carbide, pyrolytic carbon, and/or diamond-like carbon, wherein the coating is preferably implemented as fractal.

11. The stapedius muscle electrode configuration according to one of the preceding claims, wherein the two oblong elements (2a, 2b) are at least partially provided with polyurethane or silicone elastomer insulation.

12. The stapedius muscle electrode configuration according to one of the preceding claims, wherein the two oblong elements (2a, 2b) are each formed by a wire.

13. The stapedius muscle electrode configuration according to one of the preceding claims, wherein each of the two oblong elements (2a, 2b) is connected to an electrical line (5a, 5b) to carry the action currents.

14. A system made of a stapedius muscle electrode configuration according to one of Claims 1 through 13 and an oblong insertion instrument (18) for inserting the stapedius muscle electrode configuration (1") into a human body and for fastening the stapedius muscle electrode (2") on a human stapedius muscle, wherein the proximal end (17a, 17b) of at least one of the two oblong elements (2a, 2b) is detachably connected to a tip of the insertion instrument (18).

15. The system according to Claim 14, wherein the connection between the tip of the insertion element (18) and the proximal ends (17a, 17b) of the two oblong elements (2a, 2b) has a weakened zone (20), which provides an intended breakpoint.

## Revendications

1. Dispositif à électrode pour le muscle de l'étrier, destiné à détecter des courants d'action générés pendant une contraction d'un muscle de l'étrier humain, lequel dispositif comporte une électrode (2, 2', 2"), qui est adaptée à une dérivation et est destinée à être fixée à un muscle de l'étrier humain et qui comporte deux éléments allongés (2a, 2b) électroconducteurs, flexibles, élastiques, dont chacun possède une extrémité distale (4a, 4b) et une extrémité proximale (17a, 17b) et est préformé avec une forme hélicoïdale sur au moins une partie de sa longueur jusqu'à son extrémité distale (4a, 4b), de telle sorte que l'extrémité distale (4a, 4b) et un tronçon, adjacent à celle-ci, de l'élément allongé (2a, 2b) peuvent être posés au moins en partie autour du tendon (7) qui s'étend entre le muscle de l'étrier et l'étrier, et la partie hélicoïdale, guidée le long du tendon (7), peut être déplacée vers le muscle de l'étrier et peut être vissée au moins en partie dans la zone du ventre du muscle (6), adjacente au tendon (7), et/ou peut être emmanchée sur cette zone, et leurs parties hélicoïdales (3) sont agencées en étant emboîtées l'une dans l'autre, de telle sorte qu'elles tournent autour d'une ligne médiane (11) commune et peuvent être posées conjointement autour du tendon (7), peuvent être guidées le long du tendon (7) et peuvent être amenées en contact avec le muscle de l'étrier, **caractérisé en ce que** l'électrode est adaptée à une dérivation bipolaire, les deux éléments allongés (2a, 2b) étant isolés électriquement l'un par rapport à l'autre.

2. Dispositif à électrode pour le muscle de l'étrier selon la revendication 1, dans lequel l'extrémité distale (4a, 4b) de l'un au moins des deux éléments allongés (2a, 2b) est réalisée sous la forme d'une pointe de coupe (14a, 14b).

3. Dispositif à électrode pour le muscle de l'étrier selon la revendication 1 ou 2, dans lequel les parties hélicoïdales (3) des deux éléments allongés (2a, 2b) sont réalisées de telle sorte qu'elles peuvent être emmanchées sur la zone du ventre du muscle (6), adjacente au tendon (7), et, à cette occasion se dilatent radialement et poussent de manière flexible contre le ventre du muscle (6).

4. Dispositif à électrode pour le muscle de l'étrier selon l'une quelconque des revendications précédentes, dans lequel les parties hélicoïdales (3) des deux éléments allongés (2a, 2b) sont agencées en étant emboîtées l'une dans l'autre, de telle sorte qu'entre deux spires (10a, 13a) adjacentes de l'un des deux éléments allongés (2a) est agencée au moins une spire (10b, 13b) de l'autre élément allongé (2b), sachant que, de préférence, les parties hélicoïdales (3) des deux éléments allongés (2a, 2b) sont emboîtées l'une dans l'autre de telle sorte que respectivement entre deux spires (10a, 13a) adjacentes de l'un des deux éléments allongés (2a) est agencée exactement une spire (10b, 13b) de l'autre élément allongé (2b), de telle sorte que les deux parties hélicoïdales (3) sont disposées en forme de double hélice.

5. Dispositif à électrode pour le muscle de l'étrier selon l'une quelconque des revendications précédentes, dans lequel les deux éléments allongés (2a, 2b) ont une forme identique et des dimensions identiques, et/ou dans lequel les extrémités distales (4a, 4b) des deux éléments allongés (2a, 2b) sont disposées de manière directement adjacente l'une à l'autre et forment conjointement l'extrémité distale de l'électrode (2, 2', 2").

6. Dispositif à électrode pour le muscle de l'étrier selon l'une quelconque des revendications précédentes, dans lequel les parties hélicoïdales (3) des deux éléments allongés (2a, 2b) ont le même diamètre de spire au niveau de chaque position le long de la ligne médiane (11) commune, et/ou dans lequel les parties hélicoïdales (3) des deux éléments allongés (2a, 2b) sont décalées l'une par rapport à l'autre le long de la ligne médiane (11) commune sur une distance inférieure à une distance entre les spires d'au moins l'une des deux parties hélicoïdales (3).

7. Dispositif à électrode pour le muscle de l'étrier selon l'une quelconque des revendications précédentes, dans lequel les deux éléments allongés (2a, 2b) ont un diamètre de 20 à 300 µm, en particulier un diamètre de 50 à 180 µm.

8. Dispositif à électrode pour le muscle de l'étrier selon l'une quelconque des revendications précédentes, dans lequel la section des deux éléments allongés (2a, 2b) est circulaire, semi-circulaire, ovale, carrée, carrée avec des coins arrondis, rectangulaire ou rectangulaire avec des coins arrondis, et/ou dans lequel les deux éléments allongés (2a, 2b) ont une résistance à la flexion axiale de 5 à 60 Nmm², sachant qu'au moins l'un des deux éléments allongés (2a, 2b) est réalisé de préférence de telle sorte que la résistance à la flexion axiale diminue à partir de l'extrémité proximale (17a, 17b) de l'élément allongé (2a, 2b) vers l'extrémité distale (4a, 4b) de l'élément allongé (2a, 2b).

9. Dispositif à électrode pour le muscle de l'étrier selon l'une quelconque des revendications précédentes, dans lequel les parties hélicoïdales (3) des deux éléments allongés (2a, 2b) ont une section avec un diamètre de 80 à 800 µm, et/ou dans lequel les parties hélicoïdales (3) des deux éléments allongés (2a, 2b) ont, dans la direction de leur extension, une longueur de 0,1 à 3 mm, et/ou dans lequel le nombre de spires des parties hélicoïdales (3) des deux éléments allongés (2a, 2b) est de 1/4 à six.

10. Dispositif à électrode pour le muscle de l'étrier selon l'une quelconque des revendications précédentes, dans lequel les deux éléments allongés (2a, 2b) sont réalisés en acier inoxydable, dans un alliage CrCoMo, un alliage NiTi, en platine, un alliage PtIr, en argent, en or, en palladium, en tantale ou en titane ou dans un alliage de ceux-ci, et/ou dans lequel les deux éléments allongés (2a, 2b) sont revêtus au moins en partie avec du carbure de silicium, du carbone pyrolytique et/ou du carbone de type diamant, le revêtement étant réalisé de préférence selon une forme fractale.

11. Dispositif à électrode pour le muscle de l'étrier selon l'une quelconque des revendications précédentes, dans lequel les deux éléments allongés (2a, 2b) sont munis au moins en partie d'une isolation en polyuréthanne ou en élastomère de silicone.

12. Dispositif à électrode pour le muscle de l'étrier selon l'une quelconque des revendications précédentes, dans lequel les deux éléments allongés (2a, 2b) sont formés chacun par un fil.

13. Dispositif à électrode pour le muscle de l'étrier selon l'une quelconque des revendications précédentes, dans lequel chacun des deux éléments allongés (2a, 2b) est relié à une ligne électrique (5a, 5b) pour la dérivation des courants d'action.

14. Système formé par un dispositif à électrode pour le muscle de l'étrier selon l'une des revendications 1 à 13 et un instrument d'introduction (18) allongé, pour introduire le dispositif à électrode (1") dans un corps humain et pour fixer l'électrode (2") contre un muscle de l'étrier humain, l'extrémité proximale (17a, 17b) d'au moins un des deux éléments allongés (2a, 2b) étant assemblée de manière amovible à une pointe de l'instrument d'introduction (18).

15. Système selon la revendication 14, dans lequel l'assemblage entre la pointe de l'instrument d'introduction (18) et les extrémités proximales (17a, 17b) des deux éléments allongés (2a, 2b) comporte une zone affaiblie (20), qui forme une zone destinée à la rupture.
